# FASCICULE DE BREVET EUROPEEN

(11) **EP 1 033 126 B1**
(45) Date de publication et mention de la délivrance du brevet: **30.06.2004**
(21) Numéro de dépôt: 00400242.4
(22) Date de dépôt: 31.01.2000
(51) Int. Cl.: A61K 7/035, A61K 7/48

(54) **Composition cosmétique sous forme de poudre comprenant un ester d'acide gras ou d'alcohol gras**
Kosmetische Pulverzusammensetzung enthaltend einen Fettsäureester oder Fettalkoholester
Powder cosmetic composition containing a fatty acid ester or fatty alcohol ester

(30) Priorité: 04.03.1999 FR 9902705
(43) Date de publication de la demande: 06.09.2000
(73) Titulaire: L'OREAL, 75008 Paris (FR)
(72) Inventeur: Robert, Valérie, 75012 Paris (FR)
(74) Mandataire: Brédeville, Odile Marie

(56) Documents cités:
- EP-A- 0 001 471
- EP-A- 0 132 631
- EP-A- 0 194 887
- EP-A- 0 651 990
- EP-A- 0 792 633
- WO-A-94/21223
- WO-A-95/25503
- GB-A- 1 357 731
- DATABASE WPI Week 199127 Derwent Publications Ltd., London, GB; AN 1991-196389 XP002123433 & JP 03 120207 A (EARTH SEIYAKU KK), 22 mai 1991 (1991-05-22)

## Description

La présente invention a pour objet une composition cosmétique sous forme de poudre, par exemple libre, compactée, pressée ou encore coulée, comprenant un ester particulier.

Il est connu d'utiliser dans la formulation des poudres de maquillage, d'une part, une phase particulaire comportant notamment des pigments et des charges et d'autre part, une phase grasse au titre de liant comprenant des corps gras, destinée à conférer au produit fini une certaine densité, à donner une douceur et une propriété émolliente au produit de maquillage et à favoriser son adhérence sur la peau.

L'élaboration des agents liants dans les poudres, en particulier dans les poudres compactes, soulève de nombreuses difficultés car la composition finale doit être suffisamment homogène et compacte pour présenter une bonne aptitude au prélèvement et pour éviter par ailleurs une fragmentation provoquée notamment par des chocs.

Il est possible d'utiliser comme liant un mélange d'huiles minérales et végétales associées à des esters. Cependant, les produits contenant ces esters peuvent mener à des produits manquant de douceur, voire à une mauvaise dispersion de la phase pigmentaire.

Il est également possible d'utiliser des huiles de silicones qui peuvent apporter du glissant, de la douceur, de la facilité d'étalement mais les propriétés de tenue du maquillage et de résistance au choc sont médiocres.

Enfin, certaines huiles perfluorées, en particulier les perfluoropolyéthers, sont bien connues comme apportant de la douceur aux compositions cosmétiques, mais le formulateur se trouve alors confronté à des problèmes d'insolubilité de ces huiles dans les huiles hydrocarbonées usuelles ou les huiles de silicones, cette insolubilité entraînant des problèmes d'instabilité de la composition cosmétique. Par ailleurs, les compositions comprenant des huiles perfluorées présentent de médiocres propriétés de résistance au choc. Certaines de ces huiles peuvent également présenter l'inconvénient d'une mauvaise dispersion de la phase pigmentaire: la teinte apportée par le pigment est relativement peu intense, pouvant provoquer un « effet blanc », ce qui n'est pas souhaité, esthétiquement parlant.

Par ailleurs, il arrive que les compositions sous forme de poudre soient sujettes au phénomène de migration. On a en effet constaté que certaines compositions avaient tendance à se propager à l'intérieur des ridules et/ou des rides de la peau, dans le cas des poudres de visage; dans les plis de la paupière, dans le cas des fards à paupières. On a également constaté, dans le cas notamment des fards à paupières, l'apparition de stries dans le maquillage, générées par les mouvements des paupières. De telles compositions présentent une tenue non satisfaisante.

Tous ces phénomènes engendrent un effet inesthétique que l'on souhaite bien évidemment éviter.

La Demanderesse a trouvé de manière inattendue que l'utilisation comme liant de poudre d'un ester huileux particulier, constitué par des acides gras ou alcools gras saturés et ramifiés en C₂₄ à C₂₈, permettait l'obtention d'une poudre, qui non seulement présente d'excellentes propriétés cosmétiques mais qui de plus présente une tenue améliorée.

L'invention a donc pour objet une composition cosmétique selon la revendication 1.

Les compositions sous forme de poudre ainsi obtenues présentent une excellente tenue. Elles ne transfèrent pas et ne migrent pas non plus dans les plis de la peau. Elles présentent également une excellente dispersion des pigments. La composition obtenue est très homogène et elle le reste même après application sur la peau, et ce pendant plusieurs heures.

Les compositions selon l'invention présentent également d'excellentes propriétés cosmétiques : elles adhèrent suffisamment à la peau mais pas trop, elles sont très douces, elles s'appliquent facilement.

Par ailleurs, ces compositions sont faciles à compacter, elles se délitent bien, elles présentent une bonne dureté. En particulier, elles présentent une bonne cohésion du produit dans la coupelle, tout en permettant un délitage du produit satisfaisant. Dans le cas des poudres compactées par exemple, elles présentent une remarquable résistance aux chutes et un pourcentage de perte de produit après chute très faible.

La présente invention a encore pour objet un procédé cosmétique de maquillage ou de soin de la peau, en particulier du corps, ou des muqueuses (intérieurs des paupières inférieures) des êtres humains, comprenant l'application sur la peau, le corps ou les muqueuses de la composition telle que définie ci-dessus.

La présente invention a encore pour objet l'utilisation selon la revendication 22.

L'invention a encore pour objet l'utilisation selon la revendication 23.

La présente invention a aussi pour objet l'utilisation selon la revendication 24.

Les compositions selon l'invention trouvent notamment une application particulièrement intéressante dans le domaine du maquillage et/ou du soin de la peau et des muqueuses. On entend notamment par muqueuse, la partie interne de la paupière inférieure. Ainsi, l'invention trouve une application toute particulière dans le domaine des produits de maquillage du visage et de la peau, tels que les fards à paupières, les poudres du visage et du corps, les fonds de teint, les anticemes, les produits de maquillage du corps.

D'autres caractéristiques, aspects et avantages de la présente invention apparaîtront à la lecture de la description détaillée qui va suivre.

Les compositions de l'invention sont des poudres cosmétiques : elles comprennent généralement au moins 70% en poids, de préférence de 77 à 99,9% en poids, par rapport au poids total de la composition, de phase particulaire ou pulvérulente. Elles comprennent également une phase grasse à titre de liant comprenant des corps gras et qui facilite l'adhésion sur la peau des composés pulvérulents ainsi que leur cohésion entre eux au sein de la composition finale.

Cette phase grasse peut représenter jusqu'à 30% en poids, de préférence de 0,1 à 23% en poids, et préférentiellement encore de 3 à 20% en poids, par rapport au poids total de la composition.

La phase grasse des compositions selon l'invention comprend au moins un polyester d'acide gras ou d'alcool gras, la chaîne carbonée de l'acide ou alcool gras étant saturée et ramifiée et contenant 24 à 28 atomes de carbone, l'ester comportant au moins 2 chaînes ramifiées en C₂₄ à C₂₈.

Par polyester, on entend au sens de la présente invention, un composé comprenant plus d'une fonction ester comme par exemple les diesters, les triesters, les tétraesters, etc... De préférence, l'ester selon l'invention est choisi parmi les polyesters. De préférence, l'ester selon l'invention comporte au moins 2 chaînes ramifiées en C₂₄ à C₂₈. Le mot ramifié signifie au moins une chaîne pendante hydrocarbonée comportant notamment de 1 à 14 atomes de carbone.

L'ester de l'invention comporte donc un reste d'alcool gras ou acide gras saturé en C₂₄ à C₂₈ notamment du type respectivement alcool gras de Guerbet de formule (a) ou acide gras de Guerbet de formule (b) suivantes : 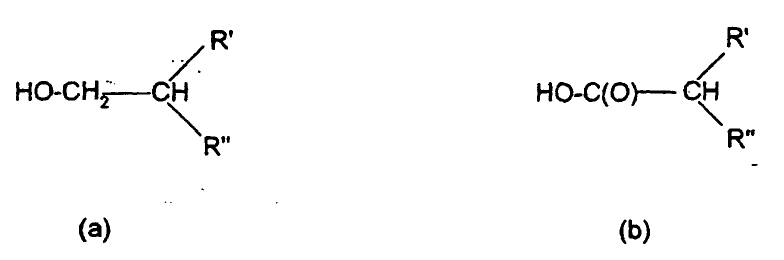 dans lesquelles R' et R" sont des radicaux alkyles saturés dont la somme des atomes de carbone va de 22 à 26. De préférence, le radical alkyle R" comprend deux atomes de carbone de moins que le radical alkyle R'.

L'ester de la composition de l'invention est, de manière préférentielle, un ester huileux liquide à température ambiante (environ 25°C), présentant un poids moléculaire élevé, c'est-à-dire ayant un nombre de carbone supérieur ou égal à 50 et notamment supérieur ou égal à 70. L'intérêt d'un produit liquide par rapport à un produit pâteux ou solide à température ambiante, réside dans le nombre plus important de ses applications et sa facilité d'utilisation. De plus, le fait que cet ester présente un poids moléculaire élevé permet l'obtention de compositions filmogènes, rémanentes à l'eau, ce qui est largement souhaité pour les produits de protection notamment solaire. Cet ester présente, entre autre, un indice de réfraction supérieur à 1,45 à 20°C et un indice d'iode ≤ 4.

Cet ester malgré son poids moléculaire élevé, n'est ni gras, ni lourd, ni collant et confère à la composition le contenant des propriétés de confort remarquable.

L'ester selon l'invention est avantageusement un ester d'acide gras ramifié en C₂₄-C₂₈ comme l'acide décyl 2-tétradécanoïque et plus spécialement un ester de polyol comme le glycérol, qui peut être un di- ou triglycéride. Préférentiellement, cet ester est un triglycéride d'acides gras ramifié en C₂₄-C₂₈ du type de Guerbet, et notamment un triglycéride d'acide gras en C₂₄ tel que l'acide décyl 2-tétradécanoïque.

De préférence, au moins une des chaînes ramifiées de l'ester contient 24 atomes de carbone.

De préférence encore, l'ester selon l'invention est choisi parmi les triglycérides d'acide gras ramifié en C₂₄, les esters d'acide gras ramifié en C₂₄ du pentaérythritol, les esters d'alcool gras ramifié en C₂₄ et de diacides et leurs mélanges.

Un triglycéride préféré est par exemple le tri(décyl 2-tétradécanoate) de glycéryle vendu sous la référence DUB TGI 24 par la société Stearinerie Dubois. Cet ester présente un indice de saponification de 140 à 150, un indice de réfraction > à 1,45 et notamment allant de 1,454 à 1,459, à 20°C, un indice d'iode ≤ 4, un indice d'hydroxyle ≤ 30, un indice d'acide ≤ 10. Son nombre de carbone est de 75.

On peut aussi utiliser les esters d'acide gras en C₂₄ du pentaérythritol comme le tétra(décyl 2-tétradécanoate) de pentaérythrityle (a 101 atomes de carbone) vendu sous la référence DUB PTI 24 par la société Stéarinerie Dubois.

Lorsque l'alcool associé à un acide gras ramifié en C₂₄ à C₂₈ est un polyol, l'estérification peut être partielle, et concerner alors 1, 2, 3 ou plus de groupes OH selon l'alcool utilisé, ou être totale.

Comme ester de l'invention comportant un reste d'alcool gras à chaîne saturée ramifiée en C₂₄ à C₂₈, on peut citer les dimérates de di(décyltétradécyle) (à 84 atomes de carbone) comme celui vendu sous la référence DUB DI 24D par la société Stéarinerie Dubois. Les dimérates sont des esters issus de diacides, ces derniers étant obtenus généralement à partir d'acide insaturé en C₆ à C₂₄ comme l'acide oléïque, linoleïque, linolénique, etc...

De préférence, l'ester selon l'invention ne présente pas de propriétés tensioactives. L'ester de l'invention peut représenter jusqu'à 100% en poids de la phase grasse de la composition selon l'invention : il peut ainsi être présent dans la composition selon l'invention à une teneur allant jusqu'à 30% en poids, par rapport au poids total de la composition. De préférence, l'ester selon l'invention est présent à une teneur allant de 0,1 à 23 % du poids de la composition, de préférence encore de de 2 à 20 %, et de façon générale, en une quantité suffisante pour conférer à la composition des propriétés de bonne cohésion et de tenue améliorée.

Outre l'ester particulier ci-dessus, la phase grasse de la composition selon l'invention peut comprendre tout autre corps gras comme des huiles et/ou des cires et/ou des corps gras pâteux.

On peut définir les composés gras pâteux à l'aide d'au moins une des propriétés physico-chimiques suivantes :
- une viscosité de 0,1 à 40 Pa.s (1 à 400 poises), de préférence 0,5 à 25 Pa.s, mesurée à 40°C avec un viscosimètre rotatif CONTRAVES TV équipé d'un mobile MS-r3 ou MS-r4 à la fréquence de 60 Hz,
- un point de fusion de 25-70°C, de préférence 25-55°C.

Les corps gras peuvent être choisis parmi les huiles et/ou cires d'origine minérale, animale ou végétale, les huiles fluorées, les esters d'acide gras et/ou leurs mélanges.

Parmi les huiles utilisables, on peut citer l'huile de vison, l'huile de tortue, l'huile de soja, l'huile de pépins de raisin, l'huile de sésame, l'huile de maïs, l'huile de colza, l'huile de tournesol, l'huile de coton, l'huile d'avocat, l'huile d'olive, l'huile de ricin, l'huile de jojoba, l'huile d'arachide ; les huiles d'hydrocarbures, telles que les huiles de paraffine, le squalane, la vaseline ; les esters gras, tels que le myristate d'isopropyle, le palmitate d'isopropyle, le stéarate de butyle, le laurate d'hexyle, l'isononate d'isononyle, le palmitate de 2-éthyl-hexyle, le laurate de 2-hexyl-décyle, le palmitate de 2-octyl-décyle, le myristate ou le lactate de 2-octyl-dodécyle, le succinate de 2-diéthyl-hexyle, le malate de diisostéaryle, le triisostéarate de glycérine ou de diglycérine ; les huiles perfluorées ; les acides gras supérieurs tels que l'acide myristique, l'acide palmitique, l'acide stéarique, l'acide béhénique, l'acide oléique, l'acide linoléique, l'acide linolénique ou l'acide isostéarique ; les alcools gras supérieurs tels que le cétanol, l'alcool stéarylique ou l'alcool oléique, et/ou leurs mélanges.

Parmi les cires utilisables, on peut citer les cires d'abeille, les cires de lanoline et les cires d'insectes de Chine ; les cires de Carnauba, de Candelila, d'ouricurry, les cires de fibres de liège, les cires de canne à sucre, les cires du Japon, les cires de jojoba hydrogénées et les huiles hydrogénées telles que l'huile de tournesol hydrogénée, l'huile de ricin hydrogénée, l'huile de coprah hydrogénée et la lanoline hydrogénée ; les paraffines, les cires microcristallines, les cires de Montan et les ozokérites ; les cires de polyéthylène, les cires obtenues par la synthèse de Fisher-Tropsch, les copolymères cireux ainsi que leurs esters, et les cires de silicone telles que les polyalcoxy et polyalkylsiloxanes et/ou leurs mélanges.

La phase grasse des compositions selon l'invention peut éventuellement comprendre une partie volatile comme par exemple des huiles volatiles.

On entend par huile volatile, tout composé susceptible de s'évaporer au contact de la peau. De préférence, on utilise des huiles dont le point éclair est suffisamment élevé pour permettre l'utilisation de ces huiles en formulation, et suffisamment bas pour obtenir l'effet évanescent souhaité. On emploie de préférence des huiles dont le point éclair est de l'ordre de 40-100°C.

Ces composés volatils peuvent être choisis en particulier parmi les huiles hydrocarbonées telles que les isoparaffines et notamment l'isododécane.

La phase grasse peut, en outre, comprendre des additifs tels que des actifs cosmétiques lipophiles et/ou des ingrédients liposolubles généralement utilisés en cosmétique comme les parfums, les filtres solaires. De préférence, ces additifs peuvent être présents en une proportion allant de 20 à 70% en poids, par rapport au poids total de la phase grasse.

Les compositions selon l'invention peuvent également comprendre des résines de silicone comprenant une combinaison des unités R₃SiO_{1/2} , R₃SiO_{2/2}, RSiO_{3/2} et SiO_{4/2}, dans lesquelles R représente l'hydrogène, un radical alkyle en C₁-C₆ ou un radical phényle.

La composition de l'invention peut comprendre, en outre, tout additif usuellement utilisé dans le domaine concerné tel que des antioxydants, des huiles essentielles, des conservateurs, des neutralisants, des tensio-actifs E/H ou H/E, des vitamines, des actifs antirides.

Bien entendu l'homme du métier veillera à choisir ce ou ces éventuels composés complémentaires, et/ou leur quantité, de manière telle que les propriétés avantageuses de la composition selon l'invention ne soient pas, ou substantiellement pas, altérées par l'adjonction envisagée.

La composition selon l'invention comprend également une phase particulaire qui peut comprendre des pigments et/ou des nacres et/ou des charges et/ou leurs mélanges habituellement utilisés dans les compositions cosmétiques.

Par pigments, il faut comprendre des particules blanches ou colorées, minérales ou organiques, insolubles dans le milieu, destinées à colorer et/ou opacifier la composition.

Les pigments peuvent être présents à raison de 0-40 % en poids, par rapport au poids total de la composition, et de préférence à raison de 2-20 %. Ils peuvent être blancs ou colorés, minéraux et/ou organiques, de taille usuelle ou nanométrique. On peut citer, parmi les pigments et les nanopigments minéraux, les dioxydes de titane, de zirconium ou de cérium, ainsi que les oxydes de zinc, de fer ou de chrome, les nanotitanes, le bleu ferrique et/ou leurs mélanges. Parmi les pigments organiques, on peut citer le noir de carbone, et les laques couramment employées pour conférer aux lèvres et à la peau un effet de maquillage, qui sont des sels de calcium, de baryum, d'aluminium ou de zirconium, de colorants acides tels que les colorants halogéno-acides, azoïques ou anthraquinoniques et/ou leurs mélanges.

Les pigments peuvent notamment être enrobés par des composés siliconés tels que des polydiméthylsiloxanes et/ou par des polymères, notamment des polyéthylènes. On peut ainsi citer les pigments SA de Maprecos ou les pigments PI de Myoshi.

Par charges, il faut comprendre des particules incolores ou blanches, minérales ou de synthèse, lamellaires ou non lamellaires, destinées à donner du corps ou de la rigidité à la composition, et/ou de la douceur, de la matité et de l'uniformité au maquillage.

Les charges, qui peuvent être présentes dans la composition à une teneur allant de 0 à 99% en poids, de préférence de 0-40 % en poids, par rapport au poids total de la composition, de préférence 2-20%, peuvent être minérales ou de synthèse, lamellaires ou non lamellaires. On peut citer le talc, le mica, la silice, le kaolin, les poudres de Nylon, de poly-β-alanine et de polyéthylène, le Téflon, la lauroyl-lysine, l'amidon, le nitrure de bore, l'oxychlorure de bismuth, les poudres de polymères de tétrafluoroéthylène, les poudres de polyméthylméthacrylate, les poudres de polyuréthanne, les poudres de polystyrène, les poudres de polyester, les microsphères creuses synthétiques telles que l'Expancel (Nobel Industrie), les microéponges comme le Polytrap (Dow Corning) et les microbilles de résine de silicone (Tospearls de la Société TOSHIBA, par exemple), les oxydes de zinc et de titane, les oxydes de zirconium ou de cérium, le carbonate de calcium précipité, le carbonate et l'hydrocarbonate de magnésium, l'hydroxyapatite, les microsphères de silice creuses (Silica Beads de la Société MAPRECOS), les microcapsules de verre ou de céramique les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium et/ou leurs mélanges.

Par nacres, il faut comprendre des particules irisées qui réfléchissent la lumière.

Les nacres peuvent être présentes dans la composition à une teneur allant de 0-60% en poids, de préférence de 0-40% en poids, de préférence encore à un taux de l'ordre de 2-20% en poids, par rapport au poids total de la composition. Parmi les nacres envisageables, on peut citer la nacre naturelle, le mica recouvert d'oxyde de titane, d'oxyde de fer, de pigment naturel ou d'oxychlorure de bismuth ainsi que le mica titane coloré, et/ou leurs mélanges.

En particulier, grâce à l'ester selon l'invention, il est possible de réaliser des poudres compactes comprenant un taux élevé de nacre. Ainsi, de préférence, lorsque le taux de nacres des compositions selon l'invention est supérieur ou égal à 30% en poids, par rapport au poids total de la composition, alors le taux d'ester selon l'invention est supérieur ou égal à 15% en poids, par rapport au poids total de la composition.

La composition selon l'invention peut se présenter sous la forme d'une poudre, par exemple compactée, libre, pressée ou encore coulée. Dans le cas d'une poudre libre par exemple, la phase grasse peut représenter jusqu'à 10% en poids, par rapport au poids total de la composition, de préférence de 1 à 5% en poids, par rapport au poids total de la composition. Pour une poudre compactée, la teneur en phase grasse peut représenter de 1 à 30% en poids, de préférence de 5 à 20% en poids, par rapport au poids total de la composition. Pour une poudre coulée, la teneur en phase grasse peut représenter de 1 à 30% en poids, de préférence de 5 à 20% en poids, par rapport au poids total de la composition.

Selon une forme préférée de réalisation de l'invention, la composition selon l'invention est une poudre compacte.

Les compositions selon l'invention sont préparées selon les méthodes connues de préparation des poudres cosmétiques, en particulier des poudres compactes.

L'invention est illustrée plus en détails dans les exemples suivants.

Dans les exemples suivants, les quantités sont données en pourcentage en poids par rapport au poids total de la composition.

### EXEMPLE 1 : comparatif

La Demanderesse a réalisé les compositions A selon l'invention et B comparative suivantes :
- Talc qsp 100%
- Mica 20 %
- BiOCl 10 %
- TᵢO₂ 2 %
- Sel métallique 3 %
- Pigments 15 %
- Nylon 20 %
- Liant 5.5%
avec

| **Composition** | **Liant** |
|---|---|
| Composition A (invention) | tri(décyl 2-tetradécanoate) de glycéryle |
| Composition B (comparatif) | polydécène-triisostéarate de glycéryle |

Ces compositions ont été préparées de la manière suivante : les composés pulvérulents sont d'abord mélangés. Le liant est ajouté et on mélange à nouveau. Le tout est tamisé puis compacté dans une coupelle.

Ces compositions peuvent par exemple se présenter sous la forme d'un fard à paupières.

La composition B comparative comprend un liant selon l'art antérieur tel que décrit dans EP 792 633. Ce liant comprend du triisostéarate de glycéryle qui est un ester de structure chimique proche du tri(décyl 2-tétradécanoate) de glycéryle.

Les compositions A et B ont été respectivement appliquées sur les deux paupières de plusieurs personnes. Les critères suivants ont ensuite été évalués :
- l'adhérence : la composition B est jugée trop adhérente.
- la douceur : 2/3 des personnes ont trouvé la composition A plus douce que la composition B.
- la facilité d'application : toutes les personnes ont trouvé que la composition A était plus facile à appliquer que la composition B.

Globalement, la composition A a été jugée comme présentant de meilleures propriétés cosmétiques que la composition B.

Ainsi, la composition selon l'invention, qui comprend un ester selon l'invention, à savoir du tri(décyl 2-tétradécanoate) de glycéryle, présente des propriétés cosmétiques améliorées par rapport à une composition comprenant un ester de structure chimique proche mais différent, le triisostéarate de glycéryle.

### EXEMPLE 2 : comparatif

Les compositions A et B de l'exemple 1 ont été appliquées respectivement sur chacune des deux paupières d'un panel de 6 personnes.

83% de ces personnes ont trouvé que :
- la composition A est plus intense après 4 heures,
- la composition A est plus homogène après 4 heures,
- la composition A reste plus mate après 4 heures.
17% des personnes n'ont pas vu de différence entre les deux produits.

Ainsi, pour 83% des personnes testées, la composition A selon l'invention présente une tenue améliorée.

### EXEMPLE 3 : comparatif

Les résistances aux chocs des compositions A et B de l'exemple 1 ont été comparées à dureté équivalente. Cette dureté a été mesurée à l'aide d'un duromètre Zwick gradué de 0 à 100 unités Shore A. On fait pénétrer l'aiguille du duromètre près du centre de la coupelle dans laquelle se trouve le produit à mesurer et on lit la dureté sur le cadran gradué. Afin d'obtenir une dureté équivalente pour les compositions A et B, la composition A a été compactée à une pression de compactage de 80 bars et la composition B a été compactée à une pression de compactage de 70 bars.

Des tests de chute ont été réalisés selon le protocole suivant : on pèse chaque échantillon préalablement compacté dans une coupelle. Puis on fait chuter 10 fois chaque échantillon d'une hauteur de 20 cm, verticalement, sur une dalle en grès. On pèse à nouveau chaque échantillon et on calcule le pourcentage de produit perdu par rapport au poids du produit initial.

Les résultats sont rassemblés dans le tableau ci-dessous :

| Composition | % de perte de produit après chute | Dureté (° Shore) |
|---|---|---|
| Composition A (invention) | 1,5% | 59 |
| Composition B (comparatif) | 2,9% | 58 |

A dureté équivalente, correspondant à un niveau de délitage cosmétiquement satisfaisant, la composition selon l'invention présente de meilleurs résultats, en test de chute et donc en résistance aux chocs, que la composition comprenant un ester non conforme à la présente invention.

### EXEMPLE 4 :

La Demanderesse a réalisé la composition C selon l'invention suivante :
- Talc qsp 100%
- Mica 10 %
- BiOCl 3 %
- TᵢO₂ 3%
- Sel métallique 2 %
- Pigments 4 %
- Nylon 10%
- Nacres 50 %
- tri(décyl 2-tétradécanoate) de glycéryle vendu sous la dénomination commerciale
   "DUB TGI 24" parla société Stéarinerie Dubois 15.4 %

Cette composition a été préparée selon la méthode de préparation de l'exemple 1. Malgré la présence de 50% de nacres, il est possible de la compacter. De plus, cette composition présente une bonne cohésion. Pour une pression de compactage de 120 bars et une dureté, mesurée comme dans l'exemple 3, de 24 Shore A, cette composition présente un pourcentage de perte de produit après chute, mesuré comme dans l'exemple 3, de 2%, ce qui est très faible pour un produit comprenant un tel taux de nacre.

La Demanderesse a également réalisé la composition D correspondant à la composition C dans laquelle les 15,4% de tri(décyl 2-tétradécanoate) de glycéryle ont été remplacés par 15,4% de silicone. Pour une pression de compactage de 120 bars et une dureté équivalente à celle de la composition C, la composition D présente un pourcentage de perte de produit après chute de 100%.

## Revendications

1. Composition cosmétique sous forme de poudre comprenant une phase particulaire et une phase grasse, **caractérisée par le fait que** la phase grasse comprend au moins un ester d'acide gras ou d'alcool gras, la chaîne carbonée de l'acide ou alcool gras étant saturée et ramifiée et contenant 24 à 28 atomes de carbone, l'ester étant un polyester et comportant au moins 2 chaînes ramifiées en C₂₄ à C₂₈.

2. Composition selon l'une quelconque des revendications précédentes, **caractérisée par le fait que** l'ester a un nombre de carbone supérieur ou égal à 50, de préférence supérieur ou égal à 70.

3. Composition selon l'une quelconque des revendications précédentes, **caractérisée par le fait qu'**au moins une des chaînes ramifiées de l'ester contient 24 atomes de carbone.

4. Composition selon l'une quelconque des revendications précédentes, **caractérisée par le fait que** l'ester est choisi parmi les triglycérides d'acide gras ramifié en C₂₄, les esters d'acide gras ramifié en C₂₄ du pentaérythritol, les esters d'alcool gras ramifié en C₂₄ et de diacides et leurs mélanges.

5. Composition selon l'une quelconque des revendications précédentes, **caractérisée par le fait que** l'ester présente un indice de réfraction supérieur à 1,45 à 20°C.

6. Composition selon l'une quelconque des revendications précédentes, **caractérisée par le fait que** l'ester présente un indice d'iode inférieur ou égal à 4.

7. Composition selon l'une quelconque des revendications précédentes, **caractérisée par le fait que** l'ester est le tri(décyl 2-tétradécanoate) de glycéryle.

8. Composition selon l'une quelconque des revendications précédentes, **caractérisée par le fait que** l'ester est présent à une teneur allant jusqu'à 30% en poids, par rapport au poids total de la composition.

9. Composition selon la revendication 8, **caractérisée par le fait que** cette teneur va de 0,1 à 23 % en poids, de préférence encore de 2 à 20 % en poids, par rapport au poids total de la composition.

10. Composition selon l'une quelconque des revendications précédentes **caractérisée par le fait que** la phase grasse comprend en outre un corps gras choisi parmi les huiles et/ou cires d'origine minérale, animale ou végétale, les huiles fluorées, les esters d'acide gras et/ou leurs mélanges.

11. Composition selon l'une quelconque des revendications précédentes **caractérisée par le fait que** la phase grasse comprend en outre une huile volatile.

12. Composition selon l'une quelconque des revendications précédentes **caractérisée par le fait que** la phase particulaire comprend des pigments choisis parmi les pigments et les nanopigments minéraux, les dioxydes de titane, de zirconium ou de cérium, les oxydes de zinc, de fer ou de chrome, les nanotitanes, le bleu ferrique, le noir de carbone, les laques telles que les sels de calcium, de baryum, d'aluminium ou de zirconium, de colorants acides tels que les colorants halogéno-acides, azoïques ou anthraquinoniques, les pigments enrobés par des composés siliconés tels que des polydiméthylsiloxanes et/ou par des polymères, notamment des polyéthylènes, et/ou leurs mélanges.

13. Composition selon la revendication 12, **caractérisée par le fait que** les pigments sont présents à une teneur allant jusqu'à 40% en poids, de préférence allant de 2 à 20% en poids, par rapport au poids total de la composition.

14. Composition selon l'une quelconque des revendications précédentes **caractérisée par le fait que** la phase particulaire comprend des charges choisies parmi le talc, le mica, la silice, le kaolin, les poudres de Nylon, de poly-β-alanine et de polyéthylène, le Téflon, la lauroyl-lysine, l'amidon, le nitrure de bore, l'oxychlorure de bismuth, les poudres de polymères de tétrafluoroéthylène, les poudres de polyméthylméthacrylate, les poudres de polyuréthanne, les poudres de polystyrène, les poudres de polyester, les microsphères creuses synthétiques, les microéponges et les microbilles de résine de silicone, les oxydes de zinc et de titane, les oxydes de zirconium ou de cérium, le carbonate de calcium précipité, le carbonate et l'hydrocarbonate de magnésium, l'hydroxyapatite, les microsphères de silice creuses, les microcapsules de verre ou de céramique ; les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium et/ou leurs mélanges.

15. Composition selon la revendication précédente, **caractérisée par le fait que** les charges sont présentes à une teneur allant de 0 à 99% en poids, de préférence de 0-40 % en poids, de préférence allant de 2 à 20% en poids, par rapport au poids total de la composition.

16. Composition selon l'une quelconque des revendications précédentes **caractérisée par le fait que** la phase particulaire comprend des nacres.

17. Composition selon la revendication précédente, **caractérisée par le fait que** les nacres sont présentes à une teneur allant de 0-60% en poids, de préférence de 0-40% en poids, de préférence allant de 2 à 20% en poids, par rapport au poids total de la composition.

18. Composition selon l'une quelconque des revendications précédentes **caractérisée par le fait qu'**elle est sous la forme d'un poudre compacte.

19. Composition selon la revendication précédente, **caractérisée par le fait qu'**elle comprend un taux de nacres supérieur ou égal à 30% en poids, par rapport au poids total de la composition et un taux d'ester supérieur ou égal à 15% en poids, par rapport au poids total de la composition.

20. Composition selon l'une quelconque des revendications précédentes, **caractérisée par le fait qu'**elle se présente sous la forme d'anticemes, de fonds de teint, de fards à paupières, de poudres du visage et du corps, de produits de maquillage du corps.

21. Procédé cosmétique de maquillage ou de soin de la peau, en particulier du corps, ou des muqueuses des êtres humains, comprenant l'application sur la peau, le corps ou les muqueuses de la composition telle que définie à l'une quelconque des revendications 1 à 20.

22. Utilisation d'au moins un polyester d'acide gras ou d'alcool gras, la chaîne carbonée de l'acide ou alcool gras étant saturée et ramifiée et contenant 24 à 28 atomes de carbone, l'ester comportant au moins 2 chaînes ramifiées en C₂₄ à C₂₈, dans une composition cosmétique sous forme de poudre, dans le but d'améliorer la tenue sur la peau de ladite composition.

23. Utilisation d'au moins un polyester d'acide gras ou d'alcool gras, la chaîne carbonée de l'acide ou alcool gras étant saturée et ramifiée et contenant 24 à 28 atomes de carbone, l'ester comportant au moins 2 chaînes ramifiées en C₂₄ à C₂₈, pour la préparation d'une composition sous forme de poudre, dans le but d'améliorer la tenue sur la peau de ladite composition.

24. Utilisation d'au moins un polyester d'acide gras ou d'alcool gras, la chaîne carbonée de l'acide ou alcool gras étant saturée et ramifiée et contenant 24 à 28 atomes de carbone, l'ester comportant au moins 2 chaînes ramifiées en C₂₄ à C₂₈, dans une composition sous forme de poudre, notamment cosmétique et plus particulièrement compacte, dans le but d'améliorer la résistance aux chocs de ladite composition.

## Patentansprüche

1. Kosmetische Zusammensetzung in Pulverform, die eine Partikelphase und eine Fettphase enthält, **dadurch gekennzeichnet, dass** die Fettphase mindestens einen Ester einer Fettsäure oder eines Fettalkohols enthält, wobei die Kohlenstoffkette der Fettsäure oder des Fettalkohols gesättigt und verzweigt ist und 24 bis 28 Kohlenstoffatome enthält und wobei der Ester ein Polyester ist und mindestens zwei verzweigte C₂₄₋₂₈-Ketten aufweist.

2. Zusammensetzung nach einem der vorhergehenden Ansprüche, **dadurch gekennzeichnet, dass** die Anzahl der Kohlenstoffatome des Esters mindestens 50 und vorzugsweise mindestens 70 beträgt.

3. Zusammensetzung nach einem der vorhergehenden Ansprüche, **dadurch gekennzeichnet, dass** mindestens eine der verzweigten Ketten des Esters 24 Kohlenstoffe besitzt.

4. Zusammensetzung nach einem der vorhergehenden Ansprüche, **dadurch gekennzeichnet, dass** der Ester unter den Triglyceriden einer verzweigten C₂₄-Fettsäure, den Pentaerythritestern einer verzweigten C₂₄-Fettsäure, den Estern von verzweigten C₂₄-Fettalkoholen und Disäuren und deren Gemischen ausgewählt ist.

5. Zusammensetzung nach einem der vorhergehenden Ansprüche, **dadurch gekennzeichnet, dass** der Ester bei 20 °C einen Brechungsindex über 1,45 aufweist.

6. Zusammensetzung nach einem der vorhergehenden Ansprüche, **dadurch gekennzeichnet, dass** der Ester eine Iodzahl von höchstens 4 aufweist.

7. Zusammensetzung nach einem der vorhergehenden Ansprüche, **dadurch gekennzeichnet, dass** es sich bei dem Ester um das Glyceryl-tri(2-decyltetradecanoat) handelt.

8. Zusammensetzung nach einem der vorhergehenden Ansprüche, **dadurch gekennzeichnet, dass** der Ester in einer Menge von bis zu 30 Gew.-%, bezogen auf das Gesamtgewicht der Zusammensetzung, enthalten ist.

9. Zusammensetzung nach Anspruch 8, **dadurch gekennzeichnet, dass** dieser Gehalt im Bereich von 0,1 bis 23 Gew.-% und vorzugsweise im Bereich von 2 bis 20 Gew.-%, bezogen auf das Gesamtgewicht der Zusammensetzung, liegt.

10. Zusammensetzung nach einem der vorhergehenden Ansprüche, **dadurch gekennzeichnet, dass** die Fettphase ferner eine Fettsubstanz enthält, die unter den Ölen und/oder Wachsen mineralischer, tierischer oder pflanzlicher Herkunft, fluorierten Ölen, Fettsäureestern und/oder deren Gemischen ausgewählt ist.

11. Zusammensetzung nach einem der vorhergehenden Ansprüche, **dadurch gekennzeichnet, dass** die Fettphase ferner ein flüchtiges Öl enthält.

12. Zusammensetzung nach einem der vorhergehenden Ansprüche, **dadurch gekennzeichnet, dass** die Partikelphase Pigmente umfasst, die unter den anorganischen Pigmenten und Nanopigmente, Dioxiden von Titan, Zirconium oder Cer, Oxiden von Zink, Eisen oder Chrom, Nanotitanen, Eisenblau, Ruß, Lacken, wie Calciumsalzen, Bariumsalzen, Aluminiumsalzen oder Zirconiumsalzen, Säurefarbstoffen, wie halogenhaltigen Säurefarbstoffen, Azofarbstoffen oder Anthrachinon-Farbstoffen, mit Siliconverbindungen, wie Polydimethylsiloxanen, und/oder Polymeren, insbesondere Polyethylenen, umhüllten Pigmenten und/oder deren Gemischen ausgewählt sind.

13. Zusammensetzung nach Anspruch 12, **dadurch gekennzeichnet, dass** die Pigmente in einer Menge von bis zu 40 Gew.-% und vorzugsweise 2 bis 20 Gew.-%, bezogen auf das Gesamtgewicht der Zusammensetzung, vorliegen.

14. Zusammensetzung nach einem der vorhergehenden Ansprüche, **dadurch gekennzeichnet, dass** die Partikelphase Füllstoffe enthält, die ausgewählt sind unter: Talk, Glimmer, Kieselsäure, Kaolin, Nylonpulver, Poly-β-Alaninpulver, Polyethylenpulver , Teflon, Lauroyllysin, Stärke, Bornitrid, Bismutoxidchlorid, Pulvern von Tetrafluorethylenpolymeren, Pulvern von Polymethylmethacrylat, Polyurethanpulvern, Polystyrolpulvern, Polyesterpulvern, Polyesterpulvern, synthetischen hohlen Mikrosphären, Mikroschwämmen, Mikrokugeln aus Siliconharz, Oxiden von Zink und Titan, Oxiden von Zirconium oder Cer, gefälltem Calciumcarbonat, Magnesiumcarbonat, Magnesiumhydrogencarbonat, Hydroxyapatit, hohlen Siliciumdioxidmikrosphären, Mikrokapseln aus Glas oder Keramik; Metallseifen, die von organischen Carbonsäuren mit 8 bis 22 Kohlenstoffatomen und vorzugsweise 12 bis 18 Kohlenstoffatomen abgeleitet sind, wie Zinkstearat, Magnesiumstearat oder Lithiumstearat, Zinklaurat, Magnesiummyristat und/oder deren Gemischen.

15. Zusammensetzung nach dem vorhergehenden Anspruch, **dadurch gekennzeichnet, dass** die Füllstoffe in einer Menge von 0 bis 99 Gew.-%, vorzugsweise 0 bis 40 Gew.-% und noch bevorzugter 2 bis 20 Gew.-%, bezogen auf das Gesamtgewicht der Zusammensetzung, enthalten sind.

16. Zusammensetzung nach einem der vorhergehenden Ansprüche, **dadurch gekennzeichnet, dass** die Partikelphase Perlglanzpigmente enthält.

17. Zusammensetzung nach dem vorhergehenden Anspruch, **dadurch gekennzeichnet, dass** die Perlglanzpigmente in einer Menge von 0 bis 60 Gew.-%, vorzugsweise 0 bis 40 Gew.-% und noch bevorzugter 2 bis 20 Gew.-%, bezogen auf das Gesamtgewicht der Zusammensetzung, enthalten sind.

18. Zusammensetzung nach einem der vorhergehenden Ansprüche, **dadurch gekennzeichnet, dass** sie als Kompaktpuder vorliegt.

19. Zusammensetzung nach dem vorhergehenden Anspruch, **dadurch gekennzeichnet, dass** sie Perlglanzpigmente in einem Gehalt von mindestens 30 Gew.-%, bezogen auf das Gesamtgewicht der Zusammensetzung, und den Ester in einer Menge von mindestens 15 Gew.-%, bezogen auf das Gesamtgewicht der Zusammensetzung, enthält.

20. Zusammensetzung nach einem der vorhergehenden Ansprüche, **dadurch gekennzeichnet, dass** sie in Form von Produkten gegen Augenringe, Make-up, Lidschatten, Pudern für das Gesicht und den Körper und Produkten zum Schminken des Körpers vorliegt.

21. Kosmetisches Verfahren zum Schminken oder zur Pflege der menschlichen Haut, insbesondere des Körpers, oder der menschlichen Schleimhäute, das umfasst, auf die Haut, den Körper oder die Schleimhäute eine Zusammensetzung nach einem der Ansprüche 1 bis 20 aufzutragen.

22. Verwendung mindestens eines Polyesters einer Fettsäure oder eines Fettalkohols, wobei die Kohlenstoffkette der Fettsäure oder des Fettalkohols gesättigt und verzweigt ist und 24 bis 28 Kohlenstoffatome enthält und wobei der Ester mindestens zwei verzweigte C₂₄₋₂₈-Ketten aufweist, in einer kosmetischen Zusammensetzung in Pulverform, um das Verhalten der Zusammensetzung auf der Haut zu verbessern.

23. Verwendung mindestens eines Polyesters einer Fettsäure oder eines Fettalkohols, wobei die Kohlenstoffkette der Fettsäure oder des Fettalkohols gesättigt und verzweigt ist und 24 bis 28 Kohlenstoffatome enthält und wobei der Ester mindestens zwei verzweigte C₂₄₋₂₈-Ketten aufweist, zur Herstellung einer Zusammensetzung in Pulverform, um das Verhalten der Zusammensetzung auf der Haut zu verbessern.

24. Verwendung mindestens eines Polyesters einer Fettsäure oder eines Fettalkohols, wobei die Kohlenstoffkette der Fettsäure oder des Fettalkohols gesättigt und verzweigt ist und 24 bis 28 Kohlenstoffatome enthält und wobei der Ester mindestens zwei verzweigte C₂₄₋₂₈-Ketten umfasst, in einer Zusammensetzung in Pulverform, insbesondere in einer kosmetischen Zusammensetzung und ganz besonders in einer kompaktierten Zusammensetzung, um die Beständigkeit der Zusammensetzung gegenüber Schlägen zu verbessern.

## Claims

1. Cosmetic composition in the form of a powder comprising a particulate phase and a fatty phase, **characterized in that** the fatty phase comprises at least one fatty acid ester or fatty alcohol ester, the carbon chain of the fatty acid or alcohol being saturated and branched and comprising 24 to 28 carbon atoms, the ester being a polyester and comprising at least 2 branched C₂₄ to C₂₈ chains.

2. Composition according to Claim 1, **characterized in that** the ester has a number of carbon atoms of greater than or equal to 50, preferably of greater than or equal to 70.

3. Composition according to either one of the preceding claims, **characterized in that** at least one of the branched chains of the ester comprises 24 carbon atoms.

4. Composition according to any one of the preceding claims, **characterized in that** the ester is chosen from triglycerides of a branched C₂₄ fatty acid, pentaerythritol esters of a branched C₂₄ fatty acid, esters of a branched C₂₄ fatty alcohol and of diacids, and their mixtures.

5. Composition according to any one of the preceding claims, **characterized in that** the ester exhibits a refractive index of greater than 1.45 at 20°C.

6. Composition according to any one of the preceding claims, **characterized in that** the ester exhibits an iodine number of less than or equal to 4.

7. Composition according to any one of the preceding claims, **characterized in that** the ester is glyceryl tri (2-decyltetradecanoate).

8. Composition according to any one of the preceding claims, **characterized in that** the ester is present at a content ranging up to 30% by weight with respect to the total weight of the composition.

9. Composition according to Claim 8, **characterized in that** this content ranges from 0.1 to 23% by weight, more preferably from 2 to 20% by weight, with respect to the total weight of the composition.

10. Composition according to any one of the preceding claims, **characterized in that** the fatty phase additionally comprises a fatty substance chosen from oils and/or waxes of mineral, animal or vegetable origin, fluorinated oils, fatty acid esters and/or their mixtures.

11. Composition according to any one of the preceding claims, **characterized in that** the fatty phase additionally comprises a volatile oil.

12. Composition according to any one of the preceding claims, **characterized in that** the particulate phase comprises pigments chosen from inorganic pigments and nanopigments, titanium, zirconium or cerium dioxides, zinc, iron or chromium oxides, titanium dioxide nanopigments, ferric blue, carbon black, lakes, such as the calcium, barium, aluminium or zirconium salts of acid dyes, such as haloacid, azo or anthraquinone dyes, pigments coated with silicone compounds, such as polydimethylsiloxanes, and/or with polymers, in particular polyethylenes, and/or their mixtures.

13. Composition according to Claim 12, **characterized in that** the pigments are present at a content ranging up to 40% by weight, preferably ranging from 2 to 20% by weight, with respect to the total weight of the composition.

14. Composition according to any one of the preceding claims, **characterized in that** the particulate phase comprises fillers chosen from talc, mica, silica, kaolin, powders formed of Nylon, of poly-β-alanine and of polyethylene, Teflon, lauroyllysine, starch, boron nitride, bismuth oxychloride, powders formed of tetrafluoroethylene polymers, poly(methyl methacrylate) powders, polyurethane powders, polystyrene powders, polyester powders, synthetic hollow microspheres, microsponges and silicone resin microbeads, zinc and titanium oxides, zirconium or cerium oxides, precipitated calcium carbonate, magnesium carbonate and hydrated magnesium carbonate, hydroxyapatite, hollow silica microspheres, glass or ceramic microcapsules, metal soaps derived from organic carboxylic acids having from 8 to 22 carbon atoms, preferably from 12 to 18 carbon atoms, for example zinc, magnesium or lithium stearate, zinc laurate or magnesium myristate, and/or their mixtures.

15. Composition according to the preceding claim, **characterized in that** the fillers are present at a content ranging from 0 to 99% by weight, preferably from 0-40% by weight, preferably ranging from 2 to 20% by weight, with respect to the total weight of the composition.

16. Composition according to any one of the preceding claims, **characterized in that** the particulate phase comprises pearlescent agents.

17. Composition according to the preceding claim, **characterized in that** the pearlescent agents are present at a content ranging from 0-60% by weight, preferably from 0-40% by weight, preferably ranging from 2 to 20% by weight, with respect to the total weight of the composition.

18. Composition according to any one of the preceding claims, **characterized in that** it is in the form of a compact powder.

19. Composition according to the preceding claim, **characterized in that** it comprises a level of pearlescent agents of greater than or equal to 30% by weight with respect to the total weight of the composition and a level of ester of greater than or equal to 15% by weight with respect to the total weight of the composition.

20. Composition according to any one of the preceding claims, **characterized in that** it is provided in the form of concealers, foundations, eyeshadows, face and body powders, or make-up products for the body.

21. Cosmetic process for making up or caring for the skin, in particular of the body, or mucous membranes of human beings comprising the application, to the skin, body or mucous membranes, of the composition as defined in any one of Claims 1 to 20.

22. Use of at least one fatty acid polyester or fatty alcohol polyester, the carbon chain of the fatty acid or alcohol being saturated and branched and comprising 24 to 28 carbon atoms, the ester comprising at least 2 branched C₂₄ to C₂₈ chains, in a cosmetic composition in the form of a powder with the aim of improving the hold on the skin of the said composition.

23. Use of at least one fatty acid polyester or fatty alcohol polyester, the carbon chain of the fatty acid or alcohol being saturated and branched and comprising 24 to 28 carbon atoms, the ester comprising at least 2 branched C₂₄ to C₂₈ chains, in the preparation of a composition in the form of a powder with the aim of improving the hold on the skin of the said composition.

24. Use of at least one fatty acid polyester or fatty alcohol polyester, the carbon chain of the fatty acid or alcohol being saturated and branched and comprising 24 to 28 carbon atoms, the ester comprising at least 2 branched C₂₄ to C₂₈ chains, in a composition in the form of a powder, especially cosmetic and more particularly compact, with the aim of improving the impact strength of the said composition.
